# EUROPEAN PATENT APPLICATION

(11) **EP 1 022 127 A2**
(43) Date of publication of application: **26.07.2000**
(21) Application number: 00100639.4
(22) Date of filing: 13.01.2000
(51) Int. Cl.: B32B 27/08, A61F 5/445

(54) **Biodegradable ostomy barrier film**

(30) Priority: 25.01.1999 EP 99101344
(71) Applicant: Cryovac, Inc., Duncan, S.C. 29334 (US)
(72) Inventor: Buongiorno, Livio, 20090 Trezzano sul Naviglio (Milan) (IT); Ciocca, Paolo, 28060 Lumellogno (Novara) (IT)
(74) Representative: De Carli, Elda

(57) **Abstract**

The invention relates to a biodegradable multilayer film material suitable for use in the manufacture of ostomy/urostomy bags and pouches comprising a gas barrier layer and two outer layers of biodegradable ester-amide copolymers. The new multilayer gas barrier film is 100% biodegradable and has improved softness and quietness characteristics.

## Description

The present invention relates to a biodegradable multilayer film material, and to its use for the production of bags and pouches for ostomy/urostomy use. Plastic film laminates having gas barrier properties are well known and widely used for current packaging requirement, as well as for use in medical applications such as the fabrication of ostomy pouches.
These generally contain a layer of PVDC, i.e. a vinylidene chloride copolymer wherein a major amount of vinylidene chloride is copolymerized with a minor amount of an unsatured monomer, typically vinyl chloride, and/or an alkyl acrylate or methyl acrylate.
While PVDC has very good baffler properties, its presence in a multilayer structure, may create environmental problems due to its chlorine content. Chlorine-free gas barrier films have therefore been developed, typically containing a gas barrier layer of ethylene-vinyl alcohol copolymer and outer layers of moisture baffler polymers, e.g. polypropylene, polyethylene etc., to prevent the degradation of the gas barrier properties of EVOH.
While the absence of a chlorine containing polymer is a step forward in the control of pollution, still from the environmental point of view, it would be highly desirable to have a plastic film which has gas barrier properties and can be easily and completely biodegraded.
In particular there exists a great need for a biodegradable gas barrier film for the manufacture of bags and pouches for medical use, such as for the manufacture of ostomy/urostomy pouches; in such a case in fact when the body wastes are discharged, the portion which directly receives and stores the body wastes could be disposed of easily and cleanly, togheter with its contents, without any separation.
For the time being a partial solution to this problem in the ostomy/urostomy area has been offered by providing structures composed of water soluble films that are laminated on the surface that would be in contact with the body wastes with a water-resistant thermoplastic material.

Thus the water soluble film would not be dissolved by the body wastes at least within a given specific time and, using a sufficiently thin layer of the water-resistant film, the pouch could be disposed off by throwing it into the toilet, where it would be dissolved or disintegrated to such a degree as not to plug up the flush toilet.

US-A-4826493 discloses a wholly biodegradable film with a layer of a polymer of hydroxybutyric acid and an outer layer of polyvinyl alcohol. This film is obtained by extruding the resins on a sacrificial layer of a thermoplastic extrudable polymer (e.g. EVA) that has then to be removed to give a fully biodegradable film. Due to the presence of the gas barrier layer, PVOH, as outer layer, good and long-lasting gas barrier properties could not be guaranteed since PVOH is a moisture susceptible polymer.

WO-A-98/25992 describes a partially biodegradable film for i.a. ostomy applications, obtained from a blend of linear ester-amide copolymers and thermoplastic polyurethane (TPU). While the ester-amide copolymers are biodegradable polymers, TPU is not. Furthermore the odour barrier properties of this film are not extremely high at the conditions of use as the gas barrier properties of these materials are very low at humid conditions.

### SUMMARY OF THE INVENTION

It has now been found that is possible to obtain, by a simple coextrusion process, a multilayer film, that can be entirely biodegraded and is endowed with very high gas barrier properties also when used in contact with liquids. A first aspect of the present invention is therefore a multilayer thermoplastic film comprising:
- a core gas barrier layer (1) comprising a polyvinyl alcohol (PVOH)
- a first outer layer (2); and
- a second outer layer(3)
wherein these outer layers, (2) and (3), that can be the same or different, comprise one or more biodegradable linear ester-amide copolymers.

### DEFINITIONS

As used herein, the term "polymer" refers to the product of a polymerization reaction, and is inclusive of homopolymers, copolymers, terpolymers, etc.

As used herein, the term "copolymer" refers to polymers formed by the polymerization reaction of at least two different monomers.

As used herein, the phrases "inner layer", "internal layer", or "intermediate layer" refers to any film layer having both of its principal surfaces directly adhered to another layer of the film.

As used herein, the phrase "outer layer" or "exterior layer" refers to any film layer having less than two of its principal surfaces directly adhered to another layer of the film. In multilayer films, there are two outer layers, each of which has a principal surface adhered to only one other layer of the multilayer film.

As used herein, the term "core" and the phrase "core layer" refers to any internal film layer which has a primary function other than serving as an adhesive or compatibilizer for adhering two layers to one another.

As used herein, the term "barrier core layer" refers to such a core layer having the primary function to serve as barrier to gasses and odours.

As used herein, the phrase "tie layer" or "adhesive layer" refers to any internal layer having the primary purpose of adhering two layers to one other.

As used herein, the term "bulk layer" refers to any layer which is present for the purpose of improving the abuse-resistance, toughness, modulus, etc., of the film.

As used herein, the term "biodegradable" when referred to a resin means that the resin is fully biologically degraded to CO₂, H₂O and biomass in a time frame comparable to paper. When referred to the end product (the film or pouch made therefrom) said term is used to mean that within 60-90 days, in an active microbial environment such as a commercial composting site, the film or pouch, becomes invisible to the unaided eye.

### DETAILED DESCRIPTION OF THE INVENTION

Polyvinyl alcohol (PVOH) is a thermoplastic material prepared by partial or complete hydrolysis of polyvinyl acetate with methanol or water.
According to a preferred embodiment of the present invention, polyvinyl alcohol polymer used for the core gas barrier layer (1), is a partially hydrolysed polyvinyl acetate.
Typically the degree of hydrolysis is comprised between about 75 and 95% by mole, preferably between about 80 and 90% by mole and even more preferably between about 84 and 88% by mole.
In the structure, according to the present invention, the polyvinyl alcohol is preferably used alone. Alternatively however it could also be blended with a minor amount of one or more other resins, preferably biodegradable, that are miscible with PVOH and do not sensibly affect the gas barrier properties thereof.
Examples of such compatible resins are aliphatic polyesters or copolyesters. The thickness of the gas-barrier layer (1) is typically up to 25 µm, generally comprised between 3 and 20 µm and preferably in the range of about 5 to 15µm.
The two outer layers (2) and (3), preferably are made from the same material. They comprise a polymer selected from the group of ester-amide copolymers containing in the block copolymer structure a combination of the polyamide segments (high crystallinity-hard segment) and the polyester segments (amorphous phase-soft segment).
The properties of these copolymers can be varied by varying the ester/amide ratio.
Suitable ester-amide copolymers consist of mainly linear ester-amide copolymers with a molecular weight above 10,000 g/mol.
The ester portion of these polymers may be obtained from linear and/or cycloaliphatic bi-functional alcohols, e.g. ethylene glycol, hexanediol, butanediol or cyclohexanedimethanol and linear and/or cycloaliphatic bi-functional acids, typically adipic acid, but also cyclohexanedicarboxylic acid, or said ester portion may be obtained starting from hydroxy acids such as hydroxybutyric acid or hydroxyvaleric acid or from the corrisponding cyclic derivates (lactones).
The amide portion may be obtained starting from linear and/or cycloaliphatic diamines such as preferably tetramethylenediamine and hexamethylenediamine and linear and/or cycloaliphatic diacids such as sebacic acid, adipic acid or cyclohexanedicarboxylic acid or from aminocarboxylic acids or the cyclic derivates thereof, typically laurolactam and caprolactam.
Available products of this class of ester-amide copolymers are e.g. those manufactured by Bayer and marketed as "BAK 1095", and those advertised by EMS as "FE 7001".
The thickness of each of the two outer surface layers is generally up to 35 µm, typically comprised between 5 and 40 µm and preferably in the range of about 10 and 35 µm.
In a preferred embodiment the outer layers (2) and (3), essentially consist of biodegradable ester-amide copolymers, so as to guarantee 100% of biodegradability to the end structure.
It is however possible also to blend these polymers with other biodegradable polymers compatible therewith, such as for instance poly-hydroxybutiryc acid or poly-hydroxy-valeric acid, still obtaining an end structure with 100% biodegradability.

Still alternatively it is possible to blend the above fully biodegradable resins with a minor proportion of a non biodegradable thermoplastic resin, such as for instance ethylene-vinyl acetate copolymer, ethylene-methacrylic acid copolymer, ethylene-alkyl-methacrylate copolymer, polyesters or copolyesters, polyurethanes, and the like resins. Using amounts up to 30%, preferably up to 20%, more preferably up to 10%, and even more preferably up to 5% by weight over the total weight of the layer, of such a non biodegradable resin, still it would be possible to obtain an end structure that after 60-90 days in an active microbial environment becomes invisible to the unaided eye.
The major proportion of fully biodegradable polymer will in fact be converted into CO₂, H₂O and biomass in these conditions leaving the minor proportion of non-degradable polymer, that was thoroughly blended with the biodegradable resin at the extrusion, as small particles invisible to the unaided eye.
The structure may comprise only the above three layers or additional internal layers may be present, e.g. bulk layers, to increase the bulk of the overall structure and/or futher improve the mechanical or gas barrier properties of the film, and tie layers to increase the adhesion between the different layers.
In another preferred embodiment, the film of the invention comprises four or five layers wherein a layer (4) is positioned between the gas barrier layer (1) and one of the outer layers (2) and (3) and/or another layer (5) is positioned between the gas barrier layer (1) and the other of the outer layers (2) and (3). Preferably, said additional layers (4) and/or (5) are of ester-amide copolymeric material or comprise a major amount, generally at least 70%, preferably at least 80%, and more preferably at least 90%, of a biodegradable ester-amide copolymer as defined above for layers (2) and (3). Alternatively other biodegradable polymers, either pure or blended with minor proportions of other compatible resins, can be employed for said intermediate layers.
The overall thickness of the laminate is generally comprised between 20 and 125 µm, preferably between 35 and 110 µm and even more preferably between 50 and 100 µm.
The resins employed in the film may contain additives, as known in the field, in order to improve resin processability or film characteristics. Examples of conventional additives that can suitably be employed include antiblocking agents, slip agents, antioxidants, pigments, opacifying agents, etc...
The multilayer film may be produced using conventional extrusion techniques. The film is not stretched or oriented, at least intentionally. A slight orientation, so-called accidental orientation, may be present depending on the production method employed. Suitable methods are the hot blown method and the cast extrusion method. In this latter case a flat die is preferably employed. In general the less orientation is introduced into the film, the less noisy it will be.
Preferably the multilayer film of the present invention is obtained by coextrusion. For example by hot-blown, i.e. by coextrusion of the multilayer film through a circular die with multiple concentric slots, using the insufflation of a bubble of cold air while the polymers are still in the molten phase, or by cast coextrusion, either trough a round or a flat die, according to methods known per se in this field.
The temperature of the extruders, the feed-block and the die will depend on the particular types of polymers used (their melting points on the one hand and their thermal stability on the other hand) and can be set up by the man skilled in the art.
It is furthermore also possible, even not preferred, to prepare the film of the invention by extrusion coating or by glue lamination of preformed layers.
The multilayer gas-barrier film according to the present invention is particulary suitable for the manufacture of containers and bags intended for human drainage in medical applications because it has very good gas- and odour- barrier properties; mechanical properties that afford a good degree of protection against wear, abrasion and puncturing; quietness; softness and "feel" features; RF- as well as heat sealability; and also biodegradability.
Another object of the present invention is therefore a biodegradable flexible container, such as a bag or a pouch, intended for human drainage in medical applications made with a multilayer thermoplastic film comprising
a core gas-barrier layer (1) comprising a PVOH
a first outer layer (2), and
a second outer layer (3)
   wherein said outer layers, that may be equal or different, comprise one or more biodegradable ester-amide copolymers.
For use in this specific application the film may be embossed in order to improve the "feel" features thereof. Embossing may be obtained by passing the film through a suitably designed calender roll before the conversion thereof into flexible containers.
Once the flexible containers have been made from the biodegradable film of the invention, the surface thereof that would be in contact with the skin of the patient, may also be laminated to a non-woven film in order to improve the comfort. In particular, in order to facilitate disposal of the used container, in such a case the non-woven layer should preferably be bonded to the skin-contact surface of the container by heat sealing it only along the container peripheral edge. Before disposal of the used pouch it will then be possible to easily remove the non-woven material.

In order that the invention may be more readily understood reference is made to the following Examples and Comparative Examples, which are intended to be illustrative of the invention, but are not intended to be limiting its scope.

### EXAMPLE 1

A three-ply biodegradable film with gas and odour barrier properties suitable for ostomy/urostomy applications is provided having the following structure:
A/B/A
wherein
A is a biodegradable ester-amide copolymer here referred to as EAC1 (m.p.= 125°C d=1.16 g/cm³) manufactured by Bayer comprising about 0.1% of tan-opaque pigment, having a thickness of 20 µm.
B is a core gas barrier layer made of polyvinyl alcohol, PVOH, (Vivinol A 08.001 manufactured by Sochital), having a thickness of 10 µm.

The three-ply structure is manufactured by the hot blown method.
The end structure has a thickness of 50 µm.

### EXAMPLE 2

A five-ply biodegradable film with gas and odour barrier properties suitable for ostomy/urostomy applications is provided having the following structure:
A/C/B/C/A
wherein
A and B are the same as in the Example 1
C is a biodegradable ester-amide copolymer here referred to as EAC2 (m.p. = 135°C d=1.14 g/cm³; MI=17 g/10 min) manufactured by Bayer having a thickness of 12.5 µm.

The end structure has a thickness of 75 µm.
The five-ply structure is manufactured bly the hot blown method.

### COMPARATIVE EXAMPLE 1

A three-ply biodegradable film is provided having the following structure:
A/C/A
wherein
A and C are the same as in the Example 2, having a thickness of 20 µm and 32.5 µm respectively.
The end structure has a thickness of 75 µm and is manufactured by the hot blown method.

### COMPARATIVE EXAMPLE 2

A biodegradable monolayer film of EAC2, having a thickness of 60 µm is manufactured by the hot blown method.

Oxygen permeability in different conditions, and moisture vapor transmission rate (MVTR) of the structures of the above Example 2 and Comparative Examples 1 and 2 have been evaluated and the results are summarized in following Table 1.

Oxygen permeability is evaluated by measuring the oxygen trasmission rate by ASTM method D-3985.
The evaluation is carried out at 23 °C and under dry conditions (0% relative humidity) and under wet conditions (100% relative humidity (R.H.)). In this latter case the test is carried out after 4 days conditioning at 100% RH., obtained by contacting both sides of the film with water.
For the structures of Example 2 the barrier properties have been evaluated also at different conditioning times (7 and 24 hours) and proved to be essentially constant with time.
Moisture vapor trasmission rate (MVTR) is evaluated according to ASTM method F-1249 at 38°C and 98% R.H..

**TABLE 1**

| Structure of Example No | Overall thickness (µm) | Oxygen permeability (ml/d.m².bar) | | MVTR (g/d.m²) |
|---|---|---|---|---|
| | | 0%R.H. | 100% R.H. | |
| 2 | 75 | 1 | 336 | 380 |

| Comp.Example | | | | |
|---|---|---|---|---|
| 1 | 75 | 250 | 510 | 226 |
| 2 | 60 | 350 | 653 | 1555 |

The biodegradable multilayer structure according to the present invention showes fair odour barrier properties.

## Claims

1. A biodegradable multilayer film material comprising
a) a core barrier layer (1) of a polyvinyl alcohol (PVOH)
b) a first outer layer (2)
c) a second outer layer (3)
wherein these layers (2) and (3) that can be the same or different, comprise one or more biodegradable linear ester-amide copolymers.

2. The biodegradable multilayer film according to claim 1 characterized in that the gas barrier layer (1) comprises polyvinyl alcohol, PVOH, alone or blended with biodegradable aliphatic polyesters or copolyesters.

3. The biodegradable multilayer film according to claim 2 characterized in that said outer layers (2) and (3), comprises the same ester-amide material.

4. The biodegradable multilayer film according to claim 3 characterized in that the thickness of the core barrier layer (1) is not more than about 25 µm preferably in the range from about 3 to about 20 µm and most preferably in the range from about 5 to about 15 µm.

5. The biodegradable multilayer film according to anyone of preceding claims, characterized in that the total thickness of the biodegradable multilayer film material is from about 20 to about 125 µm , preferably from about 35 to about 110 µm and even more preferably from about 50 to about 100 µm.

6. The biodegradable multilayer film of claim 1 wherein the first outer layer (2) and the second outer layer (3) comprises at least 70%, preferably at least 80%, more preferably at least 90%, and not even more preferably at least 95% by weight over the total weight of the layer of one or more biodegradable linear ester-amide copolymers.

7. The biodegradable multilayer film according to anyone of the preceding claims characterized in that the oxygen permeability of the total multilayer film material is below 600 and preferably below 400 cm³/m².day.bar at 23°C and 100% R.H..

8. Use of biodegradable multilayer film material according to anyone of claims 1 to 7, for the manifacture of bags and pouches for ostomy/urostomy use.
